# EUROPÄISCHE PATENTSCHRIFT

(11) **EP 0 482 349 B1**
(45) Veröffentlichungstag und Bekanntmachung des Hinweises auf die Patenterteilung: **04.07.2001**
(21) Anmeldenummer: 91115834.3
(22) Anmeldetag: 18.09.1991
(51) Int. Cl.: C07D 231/14, C07D 231/16, C07D 231/56, C07D 233/90, C07D 263/34, C07D 277/34, C07D 307/68, C07D 333/38, C07D 409/12, A01N 43/50, A01N 43/76

(54) **Sulfonamide mit herbiziden Eigenschaften.**
Sulfonamides with herbicidal properties
Sulfonamides ayant des propriétés herbicides.

(30) Priorität: 20.09.1990 DE 4029753
(43) Veröffentlichungstag der Anmeldung: 29.04.1992
(73) Patentinhaber: BASF Aktiengesellschaft, 67063 Ludwigshafen (DE)
(72) Erfinder: Heistracher, Elisabeth, Dr., W-6700 Ludwigshafen (DE); Fischer, Klaus, Dr., W-6720 Speyer (DE); Mayer, Horst, Dr., W-6700 Ludwigshafen (DE); Saupe, Thomas, Dr., W-6902 Sandhausen (DE); Hamprecht, Gerhard, Dr., W-6940 Weinheim (DE); Ditrich, Klaus, Dr., W-6702 Bad Duerkheim (DE); Kuekenhoehner, Thomas, Dr., W-6737 Boehl-Iggelheim (DE); Gerber, Matthias, Dr., W-6704 Mutterstadt (DE); Walter, Helmut, Dr., W-6719 Obrigheim (DE); Westphalen, Karl-Otto, Dr., W-6720 Speyer (DE)

(56) Entgegenhaltungen:
- EP-A- 0 244 166
- EP-A- 0 269 141
- EP-A- 0 353 640
- EP-A- 0 459 244
- DE-A- 1 670 945
- ARZNEIMITTEL FORSCHUNG, vol. 24, no. 3a, March 1974, pages 363-374, Paul-Ehrlich-Gesellschaft für Chemotherapie, Aulendorf, DE; H. PLÜMPE et al.: "Isoxazolcarboxamidoalkylbenzolsulfonyl-harnstoffe, -semicarbazide und -aminopyrimidine sowie damit verwandte Verbindungen und ihre blutzuckersenkende Wirkung"
- CHEMISCHE BERICHTE, vol. 106, no. 4, April 1973, pages 1290-1302; G. SIEWERT et al.: "Hydroxylierung von 5-Alkyl-2-(benzolsulfonylamino)pyrimidinen und strukturverwandten Antidiabetika"
- FIESER AND FIESER: "reagents for organic synthesis", , WILEY-INTERSCIENCE, USA

## Beschreibung

Die vorliegende Erfindung betrifft Sulfonamide der allgemeinen Formel I 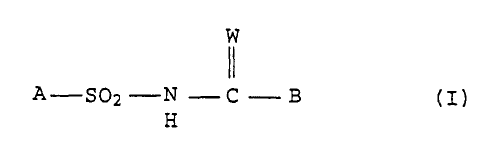 in der die Substituenten folgende Bedeutung haben:
- A: 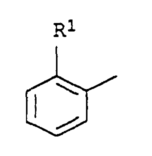
- W: Sauerstoff, Schwefel;
- B: 3-, 4- oder 5-Pyrazolyl, wobei diese genannten Reste zwei C-gebundene Reste R² und einen N-gebundenen Rest R³ tragen;
- R¹: C₁-C₆-Alkyl, welches durch ein bis fünf Halogenatome substituiert sein kann; oder COQR⁴;
- R²: Wasserstoff; C₁-C₆-Alkyl; Halogen oder zwei vicinale Reste R² bilden gemeinsam eine C₃-Kette oder eine C₄-C₆-Kette;
- R³: Wasserstoff; C₁-C₆-Alkyl;
- R⁴: C₁-C₄-Alkyl;
- Q: Sauerstoff; neue zeite sowie deren umweltverträglichen Salze.

Weiterhin betrifft die Erfindung Verfahren zur Herstellung der neuen Sulfonamide sowie herbizide und bioregulatorisch wirksame Mittel, enthaltend die Verbindungen I und deren Verwendung zur Bekämpfung unerwünschten Pflanzenwuchses.

Es ist bekannt, daß bestimmte sulfonylierte 1-Carbamoyl-2-pyrazoline herbizide und/oder wachstumsregulatorische Eigenschaften besitzen (EP-A-269 141). Desweiteren zeigen einige sulfonylierte bi- oder tricyclische Carbonsäureamide herbizide und wachstumsregulatorische Aktivitäten (EP-A-244 166). Aus EP-A 0 353 640 sind heterocyclische N-Acylsulfonamide mit herbiziden und wachstumsregulatorischen Eigenschaften bekannt. EP-A 0 459 244 beschreibt sulfonylierte Carbonsäureamide mit einem fünfgliedrigen Heterocylrest aus der Reihe Oxazolyl, Isoxazolyl, Thiazolyl, Isothiazolyl, Oxadiazolyl, Thiadiazolyl.

Der Erfindung lag die Aufgabe zugrunde, Sulfonamide mit guten herbiziden und/oder bioregulatorisch wirksamen Eigenschaften zu finden.

Entsprechend dieser Aufgabe wurden die eingangs definierten Sulfonamide I und Verfahren zu deren Herstellung gefunden. Weiterhin betrifft die Erfindung Herbizide und Mittel zur Bekämpfung des Pflanzenwuchses, die die neuen Verbindungen I enthalten, sowie ein Verfahren zur Beeinflussung und Bekämpfung von Pflanzenwuchs mit diesen Verbindungen.

Die Verbindungen der Formel I können ein oder mehrere Chrialitätszentren enthalten und liegen dann als Diastereomerengemische vor. Die Erfindung umfaßt sowohl die reinen Enantiomeren oder Diastereomeren als auch deren Gemische.

Die Verbindungen der Formel I können Salze bilden, bei denen der Wasserstoff der -SO₂-NH-Gruppe durch ein, für die Landwirtschaft, geeignetes Kation ersetzt wird. Diese Salze sind im allgemeinen Metall-, insbesondere Alkali-, Erdalkali-, gegebenenfalls alkylierte Ammonium- oder organische Aminsalze. Sie werden vorzugsweise in inerten Lösungsmitteln, wie z.B. Wasser, Methanol oder Aceton bei Temperaturen von 0-100°C hergestellt. Geeignete Basen zur Herstellung der erfindungsgemäßen Salze sind beispielsweise Alkalicarbonate, wie Kaliumcarbonat, Alkali- und Erdalkalihydroxide, Alkali- und Erdalkalialkoholate, Ammoniak oder Ethanolamin.

In den oben genannnten Definitionen bedeutet der Ausdruck "Alkyl" jeweils geradkettiges oder verzweigtes Alkyl.

Der Ausdruck "Halogen" bedeutet Fluor, Chlor, Brom oder Jod.

Die Verbindungen der Formel I sind auf vielfältige Weise analog zu bekannten Umsetzungsmethoden erhältlich. Exemplarisch seien sieben Verfahren (A bis G) im folgenden erläutert.

### Verfahren A

Man erhält Verbindungen der Formel I mit W=O, in an sich bekannter Art und Weise (M.L. Crossley, E.H. Northey, M.E. Hultquist, J. Am. Chem. Soc. 61, 2950-2955, (1939)) durch Umsetzung eines entsprechenden Sulfonamids II in einem inerten organischen Lösungsmittel in Gegenwart einer Base mit einem Säurehalogenid der Formel III gemäß dem nachfolgenden Schema: 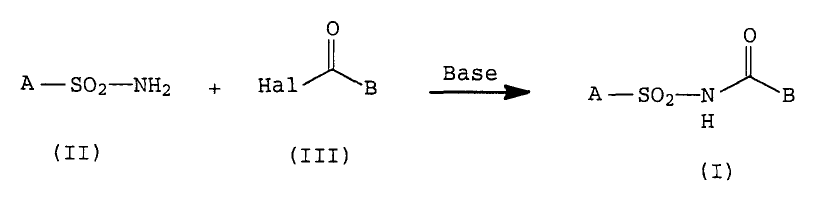 Hal in Formel III bedeutet dabei Chlor oder Brom.

Zweckmäßigerweise verwendet man für diese Umsetzungen Lösungsmittel, wie Halogenkohlenwasserstoffe, z.B. Tetrachlormethan, Chloroform, Methylenchlorid, Dichlorethan, Chlorbenzol, 1,2-Dichlorbenzol; Ether z.B. Diethylether, Methyl-tert.-butylether, Dimethoxyethan, Diethylenglykoldimethylether, Tetrahydrofuran, Dioxan; Ketone z.B. Aceton, Ethylmethylketon, Cyclohexanon; dipolare aprotische Lösungsmittel z.B. Acetonitril, N-Methylpyrrolidon; Aromaten z.B. Benzol, Toluol, Xylol, Pyridin, Chinolin oder entsprechende Gemische.

Die Umsetzung kann bei Temperaturen von 0°C bis zur Rückflußtemperatur des jeweiligen Lösungsmittels bzw. -gemisches durchgeführt werden.

Als Basen dienen dabei aromatische Stickstoffbasen, wie Pyridin, 4-Dimethylaminopyridin, Chinolin; tertiäre aliphatische Amine, wie Triethylamin, N-Ethyl-N,N-diisopropylamin und N-Methylmorpholin; bi- und tricyclische Amine, wie z.B. Diazabicycloundecen (DBU) oder Diazabicyclooctan (DABCO) sowie Hydroxide, Hydride, Alkoxide, Carbonate und Hydrogencarbonate von Alkalimetall- und Erdalkalimetallkationen, insbesondere Natriumhydroxid, Kaliumhydroxid, Natriumhydrid, Kaliumhydrid, Calciumhydrid, Lithiumhydrid, Natriummethanolat, Natriumethanolat, Kalium-tert.-butylat, Natriumcarbonat, Kaliumcarbonat, Natriumhydrogencarbonat, Kaliumhydrogencarbonat. Mitunter ist es auch nützlich, Kombinationen der oben angeführten Basen zu verwenden.

Üblicherweise setzt man die Ausgangsstoffe II und III im stöchiometrischen Verhältnis ein, jedoch kann ein Überschuß der einen oder anderen Komponente vorteilhaft sein.

Das molare Verhältnis von Sulfonamid II zu Base beträgt im allgemeinen 1:1 bis 1:3.

Die Konzentration der Edukte im Lösungsmittel beträgt im allgemeinen 0,1 bis 5,0 mol/l, bevorzugt 0,2 bis 2,0 mol/l.

Besonders bevorzugt verwendet man inerte aprotische Solventien wie Methylenchlorid, Aceton, Toluol unter Verwendung von Natriumhydrid, Natriumcarbonat, Kaliumcarbonat als Basen.

### Verfahren B

Man erhält Verbindungen der Formel I mit W=O, in an sich bekannter Weise (J.T. Drummon, G. Johnson, Tetrahedron Lett. 29, 1653-1656 (1988)) durch Umsetzung einer Verbindung der Formel IV in Gegenwart von aktivierenden Reagentien, wie 2-Chlor-1-methylpyridiniumiodid, Dicyclohexylcarbodiimid oder 1,1-Carbonyldiimidazol, und gegebenenfalls in Gegenwart einer Base, mit einer Verbindung der Formel II.

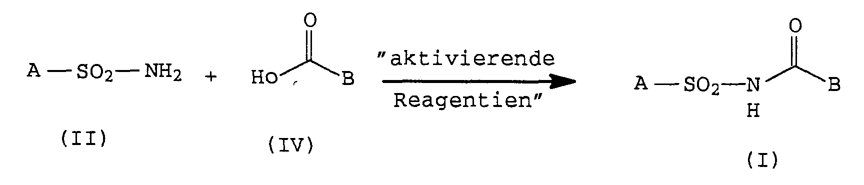

Zweckmäßigerweise wird die "aktivierte" Carbonsäure direkt ohne Zwischenisolierung gegebenenfalls in Gegenwart einer Base mit der Komponente II umgesetzt.

Die Reaktionen werden zweckmäßigerweise in Lösungsmitteln, wie Halogenkohlenwasserstoffe z.B. Chloroform, Methylenchlorid, Dichlorethan, Chlorbenzol, 1,2-Dichlorbenzol; Ether, z.B. Diethylether, Methyl-tert.-butylether, Dimethoxyethan, Diethylenglykoldimethylether, Tetrahydrofuran, Dioxan; dipolaren aprotischen Lösungsmitteln z.B. Acetonitril; Aromaten, z.B. Benzol, Toluol, Xylol oder entsprechenden Gemischen durchgeführt.

Die Umsetzungen können bei Temperaturen von -30°C bis zur Rückflußtemperatur des jeweiligen Lösungsmittels bzw. -gemisches durchgeführt werden.

Als Basen werden beispielsweise organische Stickstoffbasen, wie Pyridin, 4-Dimethylaminopyridin, Chinolin, Triethylamin, N-Ethyl-N,N-diisopropylamin, Diazabicycloundecen (DBU) etc. sowie Hydroxide, Hydride, Alkoxide, Carbonate und Hydrogencarbonate von Alkali- und Erdalkalimetallkationen, insbesondere Natriumhydroxid, Kaliumhydroxid, Natriumhydrid, Kaliumhydrid, Calciumhydrid, Natriummethanolat, Natriumethanolat, Kalium-tert.-butylat, Natriumcarbonat, Kaliumcarbonat, Natriumhydrogencarbonat, Kaliumhydrogencarbonat verwendet. Mitunter ist es von Vorteil Kombinationen der oben aufgeführten Basen zu verwenden.

Üblicherweise werden die Ausgangsstoffe II und IV, sowie das Aktivierungsreagenz im stöchiometrischen Verhältnis eingesetzt, jedoch kann ein Überschuß der einen oder anderen Komponente von Vorteil sein.

### Verfahren C

Verbindungen der Formel I, mit W=O, in an sich bekannter Weise M. Seefelder, Chem. Ber. 96, 3243-3253 (1963)) durch Umsetzung einer Verbindung der Formel V mit einer Verbindung der Formel VI erhalten werden. 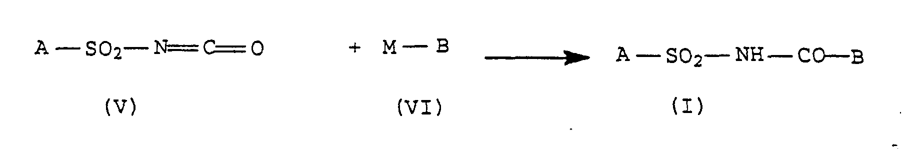 M in Formel VI bedeutet dabei Wasserstoff oder Lithium.

Zweckmäßigerweise werden inerte Lösungsmittel wie Halogenkohlenwasserstoffe, z.B. Chloroform, Methylenchlorid, Dichlorethan, Chlorbenzol, 1,2-Dichlorbenzol; Ether z.B. Tetrahydrofuran, Dioxan, Dimethoxyethan, Diethylenglycoldimethylether; Aromaten z.B. Benzol, Toluol, Xylol, Nitrobenzol oder entsprechende Gemische verwendet.

Die Umsetzungen können bei Temperaturen von -78°C bis zur Rückflußtemperatur des jeweiligen Lösungsmittels bzw. -gemisches durchgeführt werden.

Üblicherweise werden die Edukte V und VI im stöchiometrischen Verhältnis eingesetzt, jedoch kann in Einzelfällen ein Überschuß der einen oder anderen Komponente vorteilhaft sein.

### Verfahren D

Man erhält Verbindungen der Formel I, mit W=O, in an sich bekannter Weise (GB 2092 136) durch Umsetzung einer Verbindung der Formel VII mit einer Verbindung der Formel VIII in Gegenwart einer starken Base.

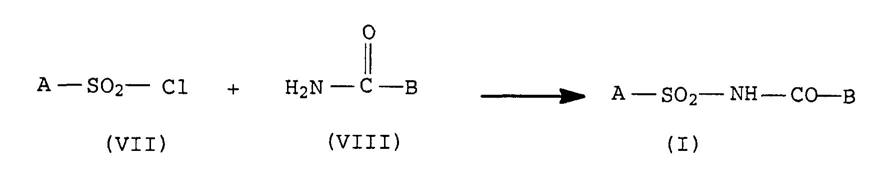

Zweckmäßigerweise werden polare, aprotische Lösungsmittel, z.B. Acetonitril, Nitromethan, Nitroethan, Nitrobenzol, Pyridin, Benzonitril, N,N-Dimethylformamid, N,N-Diethylformamid, N,N-Dimethylacetamid, Dimethylsulfoxid, N-Methylpyrrolidon, Dioxan, Tetrahydrofuran, Dimethoxyethan, Diethylenglykoldimethylether oder entsprechende Gemische verwendet.

Die Umsetzungen werden gewöhnlich in einem Temperaturbereich von -20°C bis Rückflußtemperatur des jeweiligen Lösungsmittels bzw. -gemisches durchgeführt.

Als Basen werden üblicherweise anorganische Basen, wie Oxide, Hydroxide, Hydride, Carbonate und Hydrogencarbonate sowie Alkalimetalloxide eingesetzt, insbesondere Natriumoxid, Lithiumoxid, Kaliumoxid, Natriumhydroxid, Kaliumhydroxid, Natriumhydrid, Kaliumhydrid, Calciumhydrid, Natriummethanolat, Natriumethanolat, Kaliumtertiärbutylat, Soda, Pottasche, Natriumhydrogencarbonat, Kaliumhydrogencarbonat. Mitunter kann es von Vorteil sein, Kombinationen der oben angeführten Basen zu verwenden.

In der Regel werden die Ausgangsstoffe VII und VIII, sowie die Base im stöchiometrischen Verhältnis eingesetzt, jedoch kann ein Über- oder Unterschuß der einen oder anderen Komponente von Vorteil sein.

Verfahren E

Man erhält Verbindungen der Formel I mit W=O, in an sich bekannter Art und Weise (M.M. Kremlev, V.G. Dolyuk, J. Org. Chem. (USSR) 10, 671-672 (1974)) durch Umsetzung einer Verbindung der Formel IX und einer Verbindung der Formel X mit einer Verbindung der Formel XI, 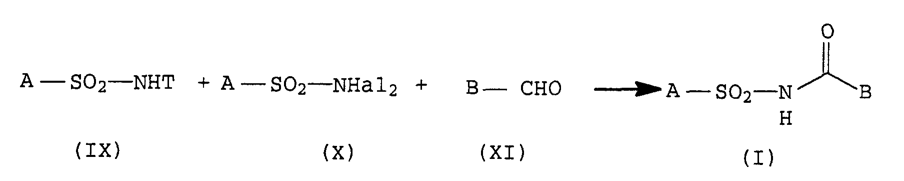 wobei T ein Alkalimetall und Hal Chlor oder Brom bedeutet.

Zweckmäßigerweise werden inerte Lösungsmittel wie Halogenkohlenwasserstoffe, z.B. Chloroform, Methylenchlorid, Dichlorethan, Tetrachlorkohlenstoff, Chlorbenzol, 1,2-Dichlorbenzol; Aromaten, z.B. Benzol, Toluol, Xylol, Nitrobenzol oder entsprechende Gemische verwendet. Die Umsetzungen können bei Temperaturen von 0°C bis zur Rückflußtemperatur des jeweiligen Lösungsmittels bzw. -gemisches durchgeführt werden.

Üblicherweise werden die Edukte im stöchiometrischen Verhältnis eingesetzt, jedoch kann in Einzelfällen ein Überschuß der einen oder anderen Komponente von Vorteil sein.

### Verfahren F

Man erhält Verbindungen der Formel I mit W=S in Analogie zu literaturbekannten Verfahren (S. Scheibye, B.S. Pederson, S.O. Lawesson, Bull. Soc. Chim. Belg. 87, 229-238 (1978); Reagent for Organic Chemistry, Fieser & Fieser, Vol. 8, S. 327) durch Umsetzung einer nach Verfahren A bis E erhaltenen Verbindung der Formel I mit der Verbindung der Formel XII (Reagent for Organic Synthesis, Fieser & Fieser, Vol. 15, S. 37) in einem inerten aprotischen 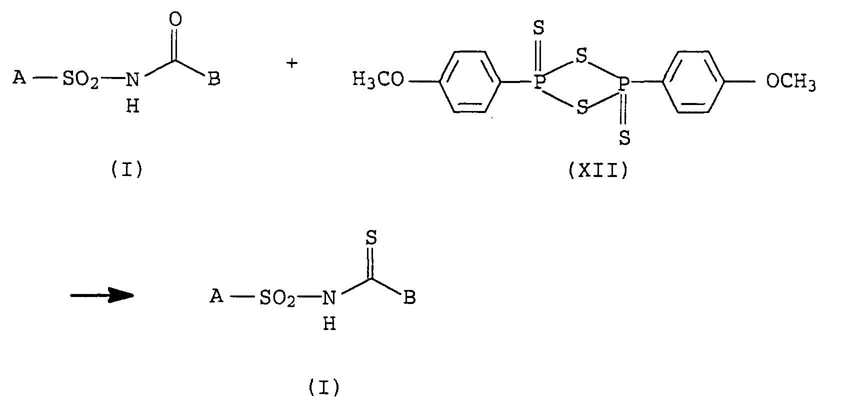 Lösungsmittel, wie z.B. Benzol, Toluol, Xylol, HMPA, Dimethoxyethan, Diethylenglykoldimethylether bzw. entsprechenden Gemischen.

Die Umsetzung kann im Temperaturbereich von etwa 0°C bis Rückflußtemperatur des jeweiligen Lösungsmittels bzw. -gemisches durchgeführt werden.

Üblicherweise setzt man die Ausgangsstoffe I (W=O) und XII im stöchiometrischen Verhältnis ein, jedoch kann ein Überschuß der einen oder anderen Komponente von Vorteil sein.

### Verfahren G

Man erhält Verbindungen der Formel I nach literaturbekannten bzw. in Analogie zu literaturbekannten Verfahren (T.L. Gilchrist, "Heterocyclic Chemistry", Pitman Publisher, London (1985)) durch Umsetzung einer nach Verfahren A bis E erhaltenen Verbindung der Formel XIII, die dadurch gekennzeichnet ist, daß der Heterocyclus B'einen C-gebundenen Substituenten, der als Abgangsgruppe fungiert, trägt, mit einem Nucleophil.

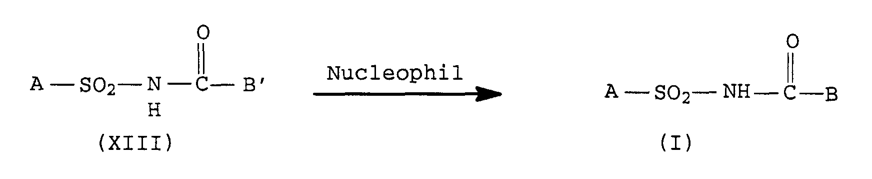

Hierbei wird die Abgangsgruppe, wie z.B. Phenolat, Chlorid, Bromid, Nitro etc. durch die "Nucleophileinheit" ersetzt.

Als Nucleophile können unter anderem Alkoholate, Thiolate, Hydride, Alkalimetallalkylen verwendet werden.

Zweckmäßigerweise werden in Anpassung an das jeweilige Nucleophil polare aprotische Lösungsmittel wie Dimethylsulfoxid (DMSO), Dimethylformamid (DMF), polare Lösungsmittel wie Alkohole, Wasser etc., inerte Lösungsmittel wie Diethylether, Methyl-tert.-butylether, Tetrahydrofuran (THF), Dioxan, Dimethoxyethan, Diethylenglykoldimethylether oder entsprechende Gemische verwendet.

Die Umsetzungen werden gewöhnlich in einem Temperaturbereich von -78°C bis Rückflußtemperatur des jeweiligen Lösungsmittels bzw. -gemisches durchgeführt.

In der Regel werden die Ausgangsstoffe im stöchiometrischen Verhältnis eingesetzt, jedoch kann ein Über- oder Unterschuß der einen oder anderen Komponente von Vorteil sein.

Die bei Verfahren A und B eingesetzten Sulfonamide der Formel II sind in vielen Fällen handelsüblich. Neue Sulfonamide der Formel II lassen sich nach allgemein bekannten Methoden darstellen (S. Pawlenko, in "Methoden der organischen Chemie", Houben-Weyl, Bd. E 11/II, S. 1098 ff., 4. Auflage, Thieme Verlag, Stuttgart 1985).

Die im Verfahren A verwendeten Säurehalogenide der Formel III lassen sich in bekannter Weise (M.F. Ansell, in "The Chemistry of acyl halides" (ed. S. Patai), S. 35ff, 1. Auflage, Interscience Publishers, London (1972)) aus den entsprechenden Carbonsäuren (IV) 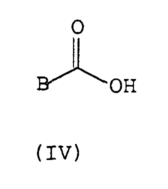 oder deren Salzen mit organischen Säurehalogeniden, wie z.B. Oxalylchlorid, Phosgen, Benzoylchlorid oder mit anorganischen Säurehalogeniden, wie z.B. POHal₃, PHal₃, PHal₅, SOCl₂, P(C₆H₅)₃Hal₂ etc. oder binären Systemen wie z.B. P(C₆H₅)₃/CCl₄ usw. darstellen.

In manchen Fällen kann der Zusatz einer geeigneten Base, insbesondere organische Stickstoffbasen, wie z.B. Pyridin, 2,6-Lutidin oder Triethylamin oder eines geeigneten Katalysators, wie z.B. Dimethylformamid oder 4-Dimethylaminopyridin zweckmäßig sein.

Die Carbonsäuren der Formel IV sind literaturbekannt oder können in Analogie zu literaturbekannten Methoden hergestellt werden (R. Sustmann, H.-G. Korth, in "Methoden der organischen Chemie", Houben-Weyl, Bd. E5/I, S. 193 ff, 4. Auflage, Thieme Verlag, Stuttgart 1985).

Die Herstellung der Sulfonylisocyanate der Formel V erfolgt nach für den Fachmann bekannten Standardverfahren ("Newer Methods of Preparative Organic Chemistry", Bd. VI, S. 223 ff, Academic Press, New York).

Die bei Verfahren C benötigten Verbindungen der Formel VI können ebenfalls nach Standardverfahren dargestellt werden.

Die bei Verfahren D eingesetzten Sulfochloride der Formel VII sind in vielen Fällen handelsüblich. Neue Sulfochloride der Formel VII lassen sich nach für den Fachmann bekannten Verfahren darstellen (S. Pawlenko, in "Methoden der organischen Chemie", Houben-Weyl, Bd. E 11/II, S. 1067 ff., 4. Auflage, Thieme Verlag, Stuttgart 1985).

Verbindungen der Formel VIII sind literaturbekannt oder können nach bekannten Methoden hergestellt werden (D. Döpp, H. Döpp, in "Methoden der organischen Chemie", Houben-Weyl, Bd. E 5/II, S. 934 ff., 4. Auflage, Thieme Verlag, Stuttgart 1985).

Die Darstellung der Salze der Formel IX erfolgt nach bekannten Standardverfahren (F. Muth, in "Methoden der organischen Chemie", Houben-Weyl, Bd. 9, S. 629 ff., 4. Auflage, Thieme Verlag, Stuttgart 1955).

Ebenso sind die Sulfonsäurehalogenide der Formel X literaturbekannt oder können auf an sich bekannter Art und Weise dargestellt werden (F. Muth, in "Methoden der organischen Chemie", Houben-Weyl, Bd. 9, S. 641 ff. 4. Auflage, Thieme Verlag, Stuttgart 1955).

Die Aldehyde der Formel XI können nach bekannten Verfahren synthetisiert werden (O. Bayer, in "Methoden der organischen Chemie", (Houben-Weyl) Bd. 7/1, 4. Auflage, Thieme Verlag, Stuttgart 1954; Bd. E3, 4. Auflage, Thieme Verlag 1983).

Im Hinblick auf die bestimmungsgemäße Verwendung sind Verbindungen der Formel I bevorzugt, in denen die Substituenten folgende Bedeutung haben:
- R¹: Halogen wie Fluor, Chlor, Brom oder Jod, insbesondere Fluor, Chlor, Brom, Cyano, Thiocyano; C₁-C₆-Alkyl wie Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, welches durch 1 bis 5 Halogenatome, insbesondere Fluor, Chlor oder Brom substituiert ist; oder COQR⁴
- R²: Wasserstoff oder einen der Reste R¹, insbesondere Fluor, Chlor, Brom, Jod, Methyl, Ethyl, Propyl, 1-Methylethyl, Butyl, 1-Methylpropyl, 2-Methylpropyl, 1,1-Dimethylethyl
oder 2 vicinale Reste R² bilden gemeinsam eine C₃-Kette wie Propylen oder eine C₄-C₆-Kette, in der eine Methylengruppe durch Sauerstoff oder eine C₁-C₄-Alkyliminoeinheit wie Methyl-, Ethyl-, Propyl- oder Butylimino ersetzt sein kann;
- R³: Wasserstoff;
gegebenenfalls substituiertes C₁-C₆-Alkyl, insbesondere C₁-C₄-Alkyl wie Methyl, Ethyl, Propyl, 1-Methylethyl, Butyl, 1-Methylpropyl, 2-Methylpropyl und 1,1-Dimethylethyl;
- R⁴: C₁-C₄-Alkyl wie Methyl, Ethyl, Propyl, 1-Methylethyl, Butyl, 1-Methylpropyl, 2-Methylpropyl und 1,1-Dimethylethyl;
- Q: Sauerstoff;

Die erfindungsgemäßen herbiziden und wachstumsregulierenden Verbindungen I bzw. die sie enthaltenden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Die Verbindungen I eignen sich allgemein zur Herstellung von direkt versprühbaren Lösungen, Emulsionen, Pasten oder Öldispersionen. Als inerte Zusatzstoffe kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel wie N,N-Dimethylformamid, Dimethylsulfoxid, N-Methylpyrrolidon oder Wasser in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten zwischen 0,1 und 95 Gew.-%, vorzugsweise zwischen 0,5 und 90 Gew.-%, Wirkstoff. Die Wirkstoffe werden dabei in einer Reinheit von 90 % bis 100 %, vorzugsweise 95 % bis 100 % (nach NMR-Spektrum) eingesetzt.

Die erfindungsgemäßen Verbindungen I können beispielsweise wie folgt formuliert werden:
I. Man vermischt 90 Gewichtsteile der Verbindung Nr. 1 mit 10 Gewichtsteilen N-Methyl-α-pyrrolidon und erhält eine Lösung, die zur Anwendung in Form kleinster Tropfen geeignet ist.
II. 20 Gewichtsteile der Verbindung Nr. 1 werden in einer Mischung gelöst, die aus 80 Gewichtsteilen Xylol, 10 Gewichtsteilen des Anlagerungsproduktes von 8 bis 10 mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 mol Ethylenoxid an 1 mol Ricinusöl besteht. Durch Ausgießen und feines Verteilen der Lösung in 100 000 Gewichtsteilen Wasser erhält man eine wäßrige Dispersion, die 0,02 Gew.-% des Wirkstoffs enthält.
III. 20 Gewichtsteile der Verbindung Nr. 1 werden in einer Mischung gelöst, die aus 40 Gewichtsteilen Cyclohexanon, 30 Gewichtsteilen Isobutanol, 20 Gewichtsteilen des Anlagerungsproduktes von 7 mol Ethylenoxid an 1 mol Isooctylphenol und 10 Gewichtsteilen des Anlagerungsproduktes von 40 mol Ethylenoxid an 1 mol Ricinusöl besteht. Durch Eingießen und feines Verteilen der Lösung in 100 000 Gewichtsteilen Wasser erhält man eine wäßrige Dispersion, die 0,02 Gew.-% des Wirkstoffs enthält.
IV. 20 Gewichtsteile des Wirkstoffs Nr. 1 werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 mol Ethylenoxid an 1 mol Ricinusöl besteht. Durch Eingießen und feines Verteilen der Lösung in 100 000 Gewichtsteilen Wasser erhält an eine wäßrige Dispersion, die 0,02 Gew.-% des Wirkstoffs enthält.
V. 20 Gewichtsteile des Wirkstoffs Nr. 1 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.-% des Wirkstoffs enthält.
VI. 3 Gewichtsteile des Wirkstoffs Nr. 1 werden mit 97 Gewichtsteilen feinteiligem Kaolin vermischt. Man erhält auf diese Weise ein Stäubemittel, das 3 Gew.-% des Wirkstoffs enthält.
VII. 30 Gewichtsteile des Wirkstoffs Nr. 1 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.
VIII. 20 Gewichtsteile des Wirkstoffs Nr. 1 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.

Die Applikation der herbiziden und wachstumsregulierenden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff bei Anwendung als Herbizide betragen je nach Bekämpfungsziel, Jahreszeit, Zielpflanzen und Wachstumsstadium 0,001 bis 3, vorzugsweise 0,01 bis 2 kg/ha aktive Substanz (a.S.).

Die Verbindungen der Formel I können praktische alle Entwicklungsstadien einer Pflanze verschiedenartig beeinflussen und werden deshalb als Wachstumsregulatoren eingesetzt. Die Wirkungsvielfalt der Pflanzenwachstumsregulatoren hängt vor allem
a) von der Pflanzenart und -sorte
b) von dem Zeitpunkt der Applikation, bezogen auf das Entwicklungsstadium der Pflanze und von der Jahreszeit,
c) von dem Applikationsort und -verfahren z.B. Samenbeize, Bodenbehandlung, Blattapplikation oder Stamminjektion bei Bäumen,
d) von klimatischen Faktoren, z.B. Temperatur, Niederschlagsmenge, außerdem auch Tageslänge und Lichtintensität,
e) von der Bodenbeschaffenheit (einschließlich Düngung),
f) von der Formulierung bzw. Anwendungsform des Wirkstoffs und schließlich
g) von den angewendeten Konzentrationen der aktiven Substanz.

Aus der Reihe der verschiedenartigen Anwendungsmöglichkeiten der erfindungsgemäßen Pflanzenwachstumsregulatoren der Formel I im Pflanzenanbau, in der Landwirtschaft und im Gartenbau, werden einige nachstehend erwähnt.

A. Mit den erfindungsgemäß verwendbaren Verbindungen läßt sich das vegetative Wachstum der Pflanzen stark hemmen, was sich insbesondere in einer Reduzierung des Längenwachstums äußert. Die behandelten Pflanzen weisen demgemäß einen gedrungenen Wuchs auf; außerdem ist eine dunklere Blattfärbung zu beobachten.

Als vorteilhaft für die Praxis erweist eine verminderte Intensität des Wachstums von Gräsern an Straßenrändern, Hecken, Kanalböschungen und auf Rasenflächen wie Park-, Sport- und Obstanlagen, Zierrasen und Flugplätzen, so daß der Arbeits- und kostenaufwendige Rasenschnitt reduziert werden kann.

Von wirtschaftlichem Interesse ist auch die Erhöhung der Standfestigkeit von lageranfälligen Kulturen wie Getreide, Mais, Sonnenblumen und Soja. Die dabei verursachte Halmverkürzung und Halmverstärkung verringern oder beseitigen die Gefahr des "Lagerns" (des Umknickens) von Pflanzen unter ungünstigen Witterungsbedingungen vor der Ernte.

Wichtig ist auch die Anwendung von Wachstumsregulatoren zur Hemmung des Längenwachstums und zur zeitlichen Veränderung des Reifeverlustes bei Baumwolle. Damit wird ein vollständig mechanisiertes Beernten dieser wichtigen Kulturpflanze ermöglicht.

Bei Obst- und anderen Bäumen lassen sich mit den Wachstumsregulatoren Schnittkosten einsparen. Außerdem kann die Alternanz von Obstbäumen durch Wachstumsregulatoren gebrochen werden.

Durch Anwendung von Wachstumsregulatoren kann auch die seitliche Verzweigung der Pflanzen vermehrt oder gehemmt werden. Daran besteht Interesse, wenn z.B. bei Tabakpflanzen die Ausbildung von Seitentrieben (Geiztrieben) zugunsten des Blattwachstums gehemmt werden soll.

Mit Wachstumsregulatoren läßt sich beispielsweise bei Winterraps auch die Frostresistenz erheblich erhöhen. Dabei werden einerseits das Längenwachstum und die Entwicklung einer zu üppigen (und dadurch besonders frostanfälligen) Blatt- bzw. Pflanzenmasse gehemmt. Andererseits werden die jungen Rapspflanzen nach der Aussaat und vor dem Einsetzen der Winterfröste trotz günstiger Wachstumsbedingungen im vegetativen Entwicklungsstadium zurückgehalten. Dadurch wird auch die Frostgefährdung solcher Pflanzen beseitigt, die zum vorzeitigen Abbau der Blühhemmung und zum Übergang in die generativen Phase neigen. Auch bei anderen Kulturen, z.B. Wintergetreide ist es vorteilhaft, wenn die Bestände durch Behandlung mit erfindungsgemäßen Verbindungen im Herbst zwar gut bestockt werden, aber nicht zu üppig in den Winter hineingehen. Dadurch kann der erhöhten Frostempfindlichkeit und - wegen der relativ geringen Blatt- bzw. Pflanzenmasse - dem Befall mit verschiedenen Krankheiten (z.B. Pilzkrankheit) vorgebeugt werden. Die Hemmung des vegetativen Wachstums ermöglicht außerdem bei vielen Kulturpflanzen eine dichtere Bepflanzung des Bodens, so daß ein Mehrertrag bezogen auf die Bodenfläche erzielt werden kann.

B. Mit den Wachstumsregulatoren lassen sich Mehrerträge sowohl an Pflanzenteilen als auch an Pflanzeninhaltsstoffen erzielen. So ist es beispielsweise möglich, das Wachstum größerer Mengen an Knospen, Blüten, Blättern, Früchten, Samenkörnern, Wurzeln und Knollen zu induzieren, den Gehalt an Zucker in Zuckerrüben, Zukkerrohr sowie Zitrusfrüchten zu erhöhen, den Proteingehalt in Getreide oder Soja zu steigern oder Gummibäume zum vermehrten Latexfluß zu stimulieren.

Dabei können die Verbindungen der Formel I Ertragssteigerungen durch Eingriffe in den pflanzlichen Stoffwechsel bzw. durch Förderung oder Hemmung des vegetativen und/oder des generativen Wachstums verursachen.

C. Mit Pflanzenwachstumsregulatoren lassen sich schließlich sowohl eine Verkürzung bzw. Verlängerung der Entwicklungsstadien als auch eine Beschleunigung bzw. Verzögerung der Reife der geernteten Pflanzenteile vor oder nach der Ernte erreichen.

Von wirtschaftlichem Interesse ist beispielsweise die Ernteerleichterung, die durch das zeitlich konzentrierte Abfallen oder Vermindern der Haftfestigkeit am Baum bei Zitrusfrüchten, Oliven oder bei anderen Arten und Sorten von Kern-, Stein- und Schalenobst ermöglicht wird. Derselbe Mechanismus, das heißt die Förderung der Ausbildung von Trenngewebe zwischen Frucht- bzw. Blatt- und Sproßteil der Pflanze ist auch für ein gut kontrollierbares Entblättern von Nutzpflanzen wie beispielsweise Baumwolle wesentlich.

D. Mit Wachstumsregulatoren kann weiterhin der Wasserverbrauch von Pflanzen reduziert werden. Dies ist besonders wichtig für landwirtschaftliche Nutzflächen, die unter einem hohen Kostenaufwand künstlich bewässert werden müssen, z.B. in ariden oder semiariden Gebieten. Durch den Einsatz der erfindungsgemäßen Substanzen läßt sich die Intensität der Bewässerung reduzieren und damit eine kostengünstigere Bewirtschaftung durchführen. Unter dem Einfluß von Wachstumsregulatoren kommt es zu einer besseren Ausnutzung des vorhandenen Wassers, weil u.a.
- die Öffnungsweite der Stomata reduziert wird
- eine dickere Epidermis und Cuticula ausgebildet werden
- die Durchwurzelung des Bodens verbessert wird und
- das Mikroklima im Pflanzenbestand durch einen kompakteren Wuchs günstig beeinflußt wird.

Die erfindungsgemäß zu verwendenden Wachstumsregulatoren der Formel I können den Kulturpflanzen sowohl vom Samen her (als Saatgutbeizmittel) als auch über den Boden, d.h. durch die Wurzel sowie - besonders bevorzugt - durch Spritzung über das Blatt zugeführt werden.

Infolge der hohen Pflanzenverträglichkeit kann die Aufwandmenge stark variiert werden.

In Anbetracht der Vielseitigkeit der Applikationsmethoden können die erfindungsgemäßen Verbindungen bzw. sie enthaltende Mittel in einer großen Zahl von Kulturpflanzen zur Beseitigung unerwünschter Pflanzen eingesetzt werden.

| Kulturenliste: | |
|---|---|
| Botanischer Name | Deutscher Name |
| Allium cepa | Küchenzwiebel |
| Ananas comosus | Ananas |
| Arachis hypogaea | Erdnuß |
| Asparagus officinalis | Spargel |
| Beta vulgaris spp. altissima | Zuckerrübe |
| Beta vulgaris spp. rapa | Futterrübe |
| Brassica napus var. napus | Raps |
| Brassica napus var. napobrassica | Kohlrübe |
| Brassica rapa var. silvestris | Rübsen |
| Camellia sinensis | Teestrauch |
| Carthamus tinctorius | Saflor - Färberdistel |
| Carya illinoinensis | Pekannußbaum |
| Citrus limon | Zitrone |
| Citrus sinensis | Apfelsine, Orange |
| Coffea arabica (Coffea canephora, Coffea liberica) | Kaffee |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum (Gossypium arboreum, Gossypium herbaceum, Gossypium vitifolium) | Baumwolle |
| Helianthus annuus | Sonnenblume |
| Hevea brasiliensis | Parakautschukbaum |
| Hordeum vulgare | Gerste |
| Humulus lupulus | Hopfen |
| Ipomoea batatas | Süßkartoffel |
| Juglans regia | Walnußbaum |
| Lens culinaris | Linse |
| Linum usitatissimum | Faserlein |
| Lycopersicon lycipersicum | Tomate |
| Malus spp. | Apfel |
| Manihot esculenta | Maniok |
| Medicago sativa | Luzerne |
| Musa spp. | Obst- und Mehlbanane |
| Nicotiana tabacum (N. rustica) | Tabak |
| Olea europaea | Ölbaum |
| Oryza sativa | Reis |
| Phaseolus lunatus | Mondbohne |
| Phaseolus vulgaris | Buschbohne |
| Picea abies | Rotfichte |
| Pinus spp. | Kiefer |
| Pisum sativum | Gartenerbse |
| Prunus avium | Süßkirsche |
| Prunus persica | Pfirsich |
| Pyrus communis | Birne |
| Ribes sylvestre | Rote Johannisbeere |
| Ricinus communis | Rizinus |
| Saccharum officinarum | Zuckerrohr |
| Secale cereale | Roggen |
| Solanum tuberosum | Kartoffel |
| Sorghum bicolor (s. vulgare) | Mohrenhirse |
| Theobroma cacao | Kakaobaum |
| Trifolium pratense | Rotklee |
| Triticum aestivum | Weizen |
| Triticum durum | Hartweizen |
| vicia faba | Pferdebohnen |
| Vitis vinifera | Weinrebe |
| Zea mays | Mais |

Zur Verbreiterung des Wirkungsspektrums und zur Erzielung synergistischer Effekte können die erfindungsgemäßen Verbindungen I mit zahlreichen Vertretern anderer herbizider oder wachstumsregulierender Wirkstoffgruppen gemischt und gemeinsam ausgebracht werden. Beispielsweise kommen als Mischungspartner Diazin, 4H-3, 1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, Chinolincarbonsäurederivate, Sulfonylharnstoffderivate, Aryloxy-, Heteroaryloxyphenoxypropionsäuren sowie deren Salze, Ester und Amide und andere in Betracht.

Außerdem kann es von Nutzen sein, die Verbindungen I allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und ölkonzentrate zugesetzt werden.

Die nachstehenden Beispiele beschreiben exemplarisch die Herstellung der erfindungsgemäßen Verbindungen.

### Beispiel 1

### N-(2-Methoxycarbonylphenylsulfonyl)-1,5-dimethylpyrazol-3-carboxamid

In 200 ml absolutem Aceton werden 10,8 g 2-Methoxycarbonylphenylsulfonamid, 8,6 g 1,5-Dimethylpyrazol-3-carbonsäurechlorid und 13,8 g Kaliumcarbonat 8 h unter Rückfluß erhitzt. Nach Abdestillieren des Lösungsmittels wird der Rückstand in Wasser aufgenommen und ein pH-Wert von 2 eingestellt. Der sich bildende Niederschlag wird abgesaugt und mit H₂O neutral gewaschen. Nach Verrühren mit Ether wird der Rückstand abgesaugt und am Vakuum getrocknet. Man erhält 11,6 g (N-2-Methoxycarbonylphenylsulfonyl )-1,5-dimethylpyrazol-3-carboxamid mit einem Schmelzpunkt von 176-177°C (Wirkstoffbeispiel Nr. 1).

Entsprechend können durch Wahl der Ausgangsstoffe und entsprechende Anpassung der Verfahrensbedingungen die nachstehend in Tabelle 1 aufgelisteten Verbindungen erhalten werden, worin die folgenden Substituenten A vorliegen:
- A1: 2-CO₂CH₃-C₆A₄
- A22: 2-CF₃-C₆H₄

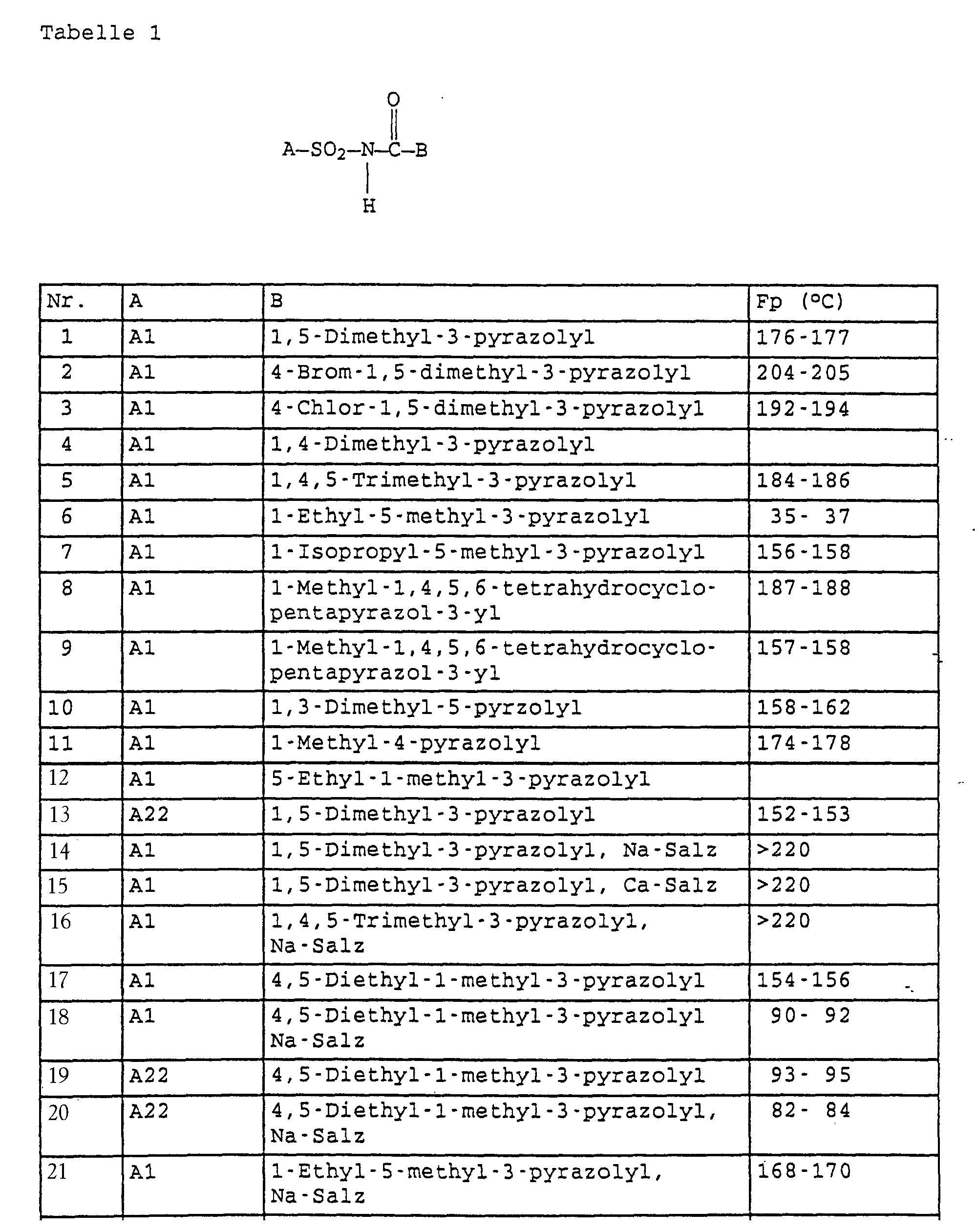

### Anwendungsbeispiele

### A. Herbizide Wirkung

Die herbizide Wirkung der Sulfonylharnstoffe der Formel I ließ sich durch Gewächshausversuche zeigen:

Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde. Die Aufwandmengen betrugen 0,125 kg/ha a.S.

Zum Zwecke der Nachauflaufbehandlung wurden die Testpflanzen je nach Wuchsform erst bei einer Wuchshöhe von 3 bis 15 cm mit den in Wasser suspendierten oder emulgierten Wirkstoffen behandelt. Die Aufwandmenge für die Nachauflaufbehandlung betrug 0,25 kg/ha a.S.

Die Pflanzen wurden artenspezifisch bei Temperaturen von 10-25°C bzw. 20-35°C gehalten. Die Versuchsperiode ersteckte sich über 2 bis 4 Wochen. Während dieser Zeit wurden die Pflanzen gepflegt und ihre Reaktion auf die einzelnen Behandlungen ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Lateinischer Name | Deutscher Name |
|---|---|
| Abutilon theophr. | Chinesischer Hanf |
| Chenopodium album | Weißer Gänsefuß |
| Triticum aestivum | Sommerweizen |

Mit 0,25 kg/ha a.S. im Nachauflaufverfahren eingesetzt, lassen sich mit dem Beispiel 1 breitblättrige unerwünschte Pflanzen sehr gut bekämpfen, bei gleichzeitiger Verträglichkeit an der Beispielkultur Weizen.

### B. Wachstumsregulierende Wirkung

Zur Bestimmung der wachstumsregulierenden Eigenschaft der Prüfsubstanzen wurden Testpflanzen auf ausreichend mit Nährstoffen versorgtem Kultursubstrat in Kunststoffgefäßen von ca. 12,5 cm Durchmesser angezogen.

Im Nachauflaufverfahren wurden die zu prüfenden Substanzen in wäßriger Aufbereitung auf die Pflanzen gesprüht. Die beobachtete wachstumsregulierende Wirkung wurde bei Versuchsende durch Wuchshöhenmessung belegt. Die so gewonnenen Meßwerte wurden zur Wuchshöhe der unbehandelten Pflanzen in Relation gesetzt. Als Vergleichssubstanz A diente 2-Chlorethyltrimethylammoniumchlorid.

Gleichlaufend zur Reduzierung des Längenwachstums stieg die Farbintensität der Blätter an. Der erhöhte Chlorophyllgehalt läßt eine ebenfalls erhöhte Photosyntheserate und damit eine erhöhte Ertragsbildung erwarten.

Die Einzeldaten sind den folgenden Tabellen B-1 und B-2 zu entnehmen.

**Tabelle B-1**

| Sommerweizen, "Ralle"; Nachauflauf-Blattbehandlung | | |
|---|---|---|
| Nr.d.chem.Beispiele | Konzentration mg a.S./Gefäß | Wuchshöhen rel. |
| unbehandelt | - | 100 |
| A | 1,5 | 82,2 |
| 1 | 1,5 | 73,1 |

**Tabelle B-2**

| Sommerweizen, "Aramir"; Nachauflauf-Blattbehandlung | | |
|---|---|---|
| Nr.d.chem.Beispiele | Konzentration mg a.S./Gefäß | Wuchshöhen rel. |
| unbehandelt | - | 100 |
| A | 1,5 | 90,7 |
| 1 | 1,5 | 69,4 |

## Patentansprüche

1. Sulfonamide der allgemeinen Formel I 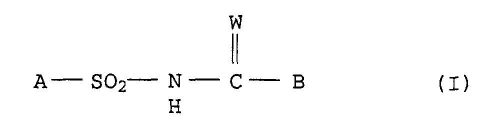 in der die Substituenten folgende Bedeutung haben:
A 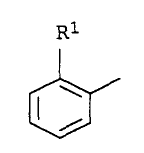
W Sauerstoff, Schwefel;
B 3-, 4- oder 5-Pyrazolyl, wobei diese genannten Reste zwei C-gebundene Reste R² und einen N-gebundenen Rest R³ tragen;
R¹ C₁-C₆-Alkyl, welches durch ein bis fünf Halogenatome substituiert sein kann;
oder
COQR⁴;
R² Wasserstoff;
C₁-C₆-Alkyl; Halogen oder zwei vicinale Reste R² bilden gemeinsam eine C₃-Kette oder eine C₄-C₆-Kette;
R³ Wasserstoff;
C₁-C₆-Alkyl;
R⁴ C₁-C₄-Alkyl;
Q Sauerstoff;
sowie deren umweltverträglichen Salze.

2. Sulfonamide der allgemeinen Formel (I) gemäß Anspruch 1 in der die Substituenten folgende Bedeutung haben:
W Sauerstoff;
R¹ COQR⁴;
B 1,5-Dimethyl-3-pyrazolyl
1,5-Dimethyl-3-pyrazolyl, Ca-Salz
1-Ethyl-5-methyl-3-pyrazolyl
1-Methyl-1,4,5,6-tetrahydrocyclopentapyrazol-3-yl
4-Chlor-1,5-dimethyl-3-pyrazolyl;
R⁴ Methyl;
Q Sauerstoff;
sowie deren umweltverträglichen Salze.

3. Sulfonamid der allgemeinen Formel (I) gemäß Anspruch 1 in der die Substituenten folgende Bedeutung haben:
W Sauerstoff;
R¹ CF₃;
B 1,5-Dimethyl-3-pyrazolyl;
sowie dessen umweltverträgliches Salz.

4. Verfahren zur Herstellung der Verbindungen der Formel I mit W=O oder deren Salze gemäß Anspruch 1, dadurch gekennzeichnet, daß man eine Verbindung der Formel II
A-SO₂NH₂ (II)
mit einer Verbindung der Formel III 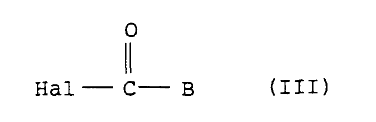 in der Hal Chlor oder Brom bedeutet, in Gegenwart einer Base in einem inerten Lösungsmittel in an sich bekannter Weise umsetzt.

5. Verfahren zur Herstellung der Verbindungen der Formel I mit W=O oder deren Salze gemäß Anspruch 1, dadurch gekennzeichnet, daß man eine Carbonsäure der Formel IV 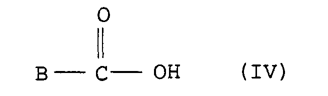 in Anwesenheit von aktivierenden Reagentien, wie 2-Chlor-1-methylpyridiniumiodid, Dicyclohexylcarbodiimid oder 1,1-Carbonyldiimidazol und gegebenenfalls in Gegenwart einer Base mit einer Verbindung der Formel II gemäß Anspruch 2 in an sich bekannter Weise umsetzt.

6. Verfahren zur Herstellung der Verbindungen der Formel I mit W=O oder deren Salze gemäß Anspruch 1, dadurch gekennzeichnet, daß man eine Verbindung der Formel V
A-SO₂-N=C=O (V)
mit einer Verbindung der Formel VI
M-B (VI),
wobei M Wasserstoff oder Lithium bedeutet, umsetzt.

7. Verfahren zur Herstellung der Verbindungen der Formel I mit W=O oder deren Salze gemäß Anspruch 1, dadurch gekennzeichnet, daß man eine Verbindung der Formel VII
A-SO₂-Cl (VII)
in Gegenwart einer starken Base mit einem Säureamid der Formel VIII 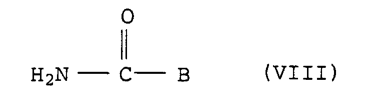 in an sich bekannter Weise umsetzt.

8. Verfahren zur Herstellung der Verbindungen der Formel I mit W=O oder deren Salze gemäß Anspruch 1, dadurch gekennzeichnet, daß man eine Verbindung der Formel IX
A-SO₂-NHT (IX)
worin T ein Alkalimetall bedeutet, mit einer Verbindung der Formel X
ASO₂NHal₂ (X)
worin Hal für Chlor oder Brom steht, und einem Aldehyd der Formel XI
B-CHO (XI)
in an sich bekannter Weise umsetzt.

9. Verfahren zur Herstellung von Sulfonamiden der allgemeinen Formel I 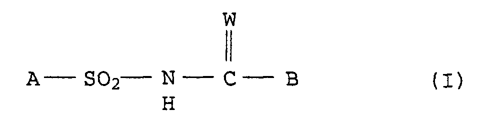 in der die Substituenten die in Anspruch 1 angegebene Bedeutung haben;
sowie deren umweltverträglichen Salze,
dadurch gekennzeichnet, daß man eine Verbindung der Formel I mit W=O mit der Verbindung der Formel XII 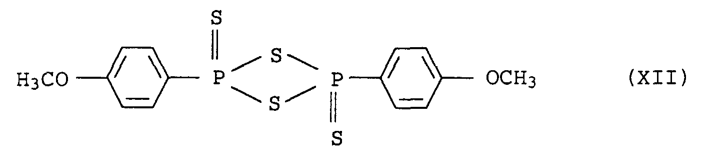 in an sich bekannter Weise umsetzt.

10. Verfahren zur Herstellung von Verbindungen der Formel I mit W=O oder deren Salze gemäß Anspruch 1, dadurch gekennzeichnet, daß man eine Verbindung der Formel XIII 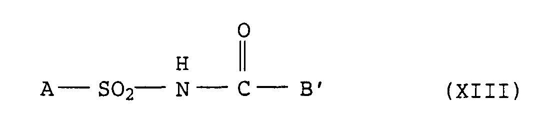 in der Heterocyclus B' einen C-gebundenen Substituenten trägt, der als Abgangsgruppe fungiert, mit einem Nucleophil in an sich bekannter Weise umsetzt.

11. Herbizides Mittel, enthaltend ein Sulfonamid der Formel I gemäß Anspruch 1 oder dessen Salz sowie übliche inerte Zusatzstoffe.

12. Mittel zur Beeinflussung des Pflanzenwuches, enthaltend ein Sulfonamid der Formel I gemäß Anspruch 1 oder dessen Salz sowie übliche inerte Zusatzstoffe.

13. Verfahren zur Bekämpfung unerwünschten Pflanzenwuches, dadurch gekennzeichnet, daß man ein Sulfonamid der Formel I, gemäß Anspruch 1 oder dessen Salz auf die Pflanzen und/oder deren Lebensraum einwirken läßt.

14. Verfahren zur Beeinflussung des Pflanzenwachstums, dadurch gekennzeichnet, daß man ein Sulfonamid der Formel I, gemäß Anspruch 1 oder dessen Salz auf die Pflanzen und/oder deren Lebensraum einwirken läßt.

## Claims

1. A sulfonamide of the formula I 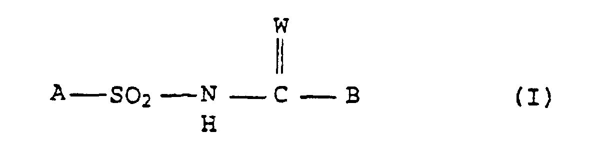 where
A is 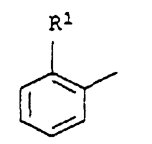
W is oxygen or sulfur;
B is 3-, 4- or 5-pyrazolyl, each disubstituted on carbon by R² and monosubstituted on nitrogen by R³;
R¹ is C₁-C₆-alkyl which can be substituted by one to five halogens;
or
COQR⁴;
R² is hydrogen;
C₁-C₆-alkyl; halogen or two vicinal R² together form a C₃ chain or a C₄-C₆ chain;
R³ is hydrogen;
C₁-C₆-alkyl;
R⁴ is C₁-C₄-alkyl;
Q is oxygen;
and the environmentally compatible salts thereof.

2. A sulfonamide of the formula I as claimed in claim 1, where
w is oxygen:
R¹ is COQR⁴;
B is 1,5-dimethyl-3-pyrazolyl
1,5-dimethyl-3-pyrazolyl, Ca salt
1-ethyl-5-methyl-3-pyrazolyl
1-methyl-1,4,5,6-tetrahydrocyclopentapyrazolyl-3-yl 4-chloro-1,5-dimethyl-3-pyrazolyl;
R⁴ is methyl;
Q is oxygen;
and the environmentally compatible salts thereof.

3. A sulfonamide of the formula I as claimed in claim 1, where
W is oxygen;
R¹ is CF₃;
B is 1,5-dimethyl-3-pyrazolyl;
and the environmentally compatible salts thereof.

4. A process for preparing a compound of the formula I with W=O or the salts thereof as claimed in claim 1, which comprises reacting a compound of the formula II
A-SO₂NH₂ (II)
with a compound of the formula III 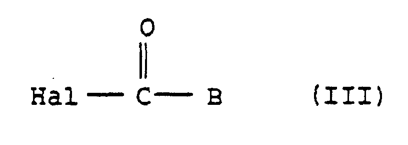 where Hal is chlorine or bromine, in the presence of a base in an inert solvent in a conventional manner.

5. A process for preparing a compound of the formula I with W=O or the salts thereof as claimed in claim 1, which comprises reacting a carboxylic acid of the formula IV 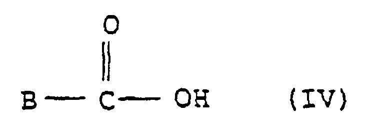 in the presence of activating reagents, such as 2-chloro-1-methylpyridinium iodide, dicyclohexylcarbodiimide or 1,1-carbonyldiimidazole and in the presence or absence of a base with a compound of the formula II as claimed in claim 2 in a conventional manner.

6. A process for preparing a compound of the formula I with W=O or the salts thereof as claimed in claim 1, which comprises reacting a compound of the formula V
A-SO₂-N=C=O (V)
with a compound of the formula VI
M-B (VI)
where M is hydrogen or lithium.

7. A process for preparing a compound of the formula I with W=O or the salts thereof as claimed in claim 1, which comprises reacting a compound of the formula VII
A-SO₂-Cl (VII)
in the presence of a strong base with an amide of the formula VIII 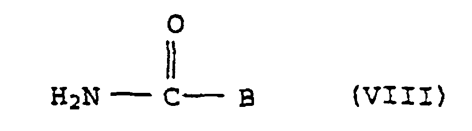 in a conventional manner.

8. A process for preparing a compound of the formula I with W=O or the salts thereof as claimed in claim 1, which comprises reacting a compound of the formula IX
A-SO₂-NHT (IX)
where T is an alkali metal, with a compound of the formula X
ASO₂NHal₂ (X)
where Hal is chlorine or bromine, and with an aldehyde of the formula XI
B-CHO (XI)
in a conventional manner.

9. A process for preparing a sulfonamide of the formula I 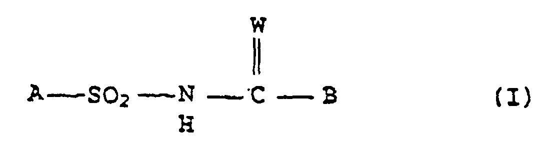 where the substituents have the meanings indicated in claim 1;
and the environmentally compatible salts thereof,
which comprises reacting a compound of the formula I with W=O with the compound of the formula XII 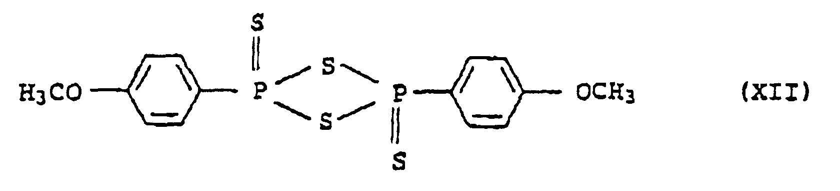 in a conventional manner.

10. A process for preparing a compound of the formula I with W=O or the salts thereof as claimed in claim 1, which comprises reacting a compound of the formula XIII 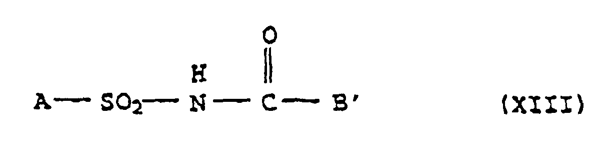 where heterocycle B' has a C-bonded substituent which acts as leaving group, with a nucleophile in a conventional manner.

11. A herbicide containing a sulfonamide of the formula I as claimed in claim 1 or the salt thereof in addition to conventional inert additives.

12. An agent for influencing plant growth, containing a sulfonamide of the formula I as claimed in claim 1 or the salt thereof in addition to conventional inert additives.

13. A process for controlling undesired plant growth, which comprises allowing a sulfonamide of the formula I as claimed in claim 1 or the salt thereof to act on the plants and/or their habitat.

14. A process for influencing plant growth, which comprises allowing a sulfonamide of the formula I as claimed in claim 1 or the salt thereof to act on the plants and/or their habitat.

## Revendications

1. Sulfonamides de formule générale I 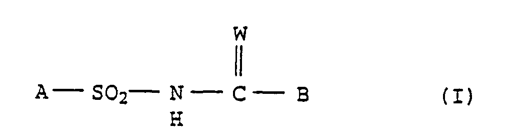 dans laquelle les symboles ont les significations suivantes :
A: 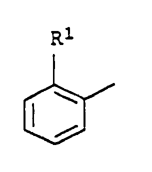
W : l'oxygène, le soufre ;
B : un groupe 3-, 4- ou 5-pyrazolyle, ces groupes pouvant porter deux substituants R² reliés au carbone et un substituant R³ relié à l'azote ;
R¹ : un groupe alkyle en C1-C6 qui peut porter un à cinq substituants halogéno ;
ou bien
COQR⁴ ;
R² : l'hydrogène ;
un groupe alkyle en C1-C6 ; un halogène ; ou bien deux substituants R² voisins forment ensemble une chaîne en C3 ou une chaîne en C4-C6 ;
R³ : l'hydrogène ;
un groupe alkyle en C1-C6 ;
R⁴ : un groupe alkyle en C1-C4 ;
Q : l'oxygène ;
et leurs sels non polluants.

2. Sulfonamides de formule générale I selon la revendication 1 pour lesquels les symboles ont les significations suivantes :
W : l'oxygène ;
R¹ : COQR⁴ ;
B : un groupe 1,5-diméthyl-3-pyrazolyle,
un groupe 1,5-diméthyl-3-pyrazolyle, sel de calcium,
un groupe 1-éthyl-5-méthyl-3-pyrazolyle,
un groupe 1-méthyl-1,4,5,6-tétrahydrocyclopentapyrazol-3-yle,
un groupe 4-chioro-1,5-diméthyl-3-pyrazolyle;
R⁴ : un groupe méthyle ;
Q : l'oxygène ;
et leurs sels non polluants.

3. Sulfonamide de formule générale I de la revendication 1 pour lequel les symboles ont les significations suivantes :
W : l'oxygène ;
R¹ : CF₃ ;
B : 1,5-diméthyl-3-pyrazolyle ;
et ses sels non polluants.

4. Procédé de préparation des composés de formule I dans laquelle W = O ou de leurs sels selon la revendication 1, caractérisé par le fait que l'on fait réagir de manière connue en soi un composé de formule II
A-SO₂NH₂ (II)
avec un composé de formule III 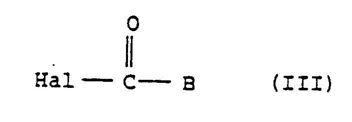 dans laquelle Hal représente le chlore ou le brome, en présence d'une base, dans un solvant inerte.

5. Procédé de préparation des composés de formule I dans laquelle W = O ou de leurs sels selon la revendication 1, caractérisé par le fait que l'on fait réagir de manière connue en soi un acide carboxylique de formule IV 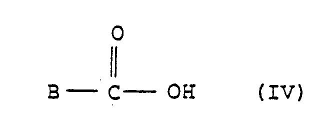 en présence de réactifs activant tels que l'iodure de 2-chloro-1-méthylpyridinium, le dicyclohexylcarbodiimide ou le 1,1-carbonyldiimidazole et le cas échéant en présence d'une base, avec un composé de formule II selon la revendication 2.

6. Procédé de préparation des composés de formule I dans laquelle W = O ou de leurs sels selon la revendication 1, caractérisé par le fait que l'on fait réagir un composé de formule V
A-SO₂-N=C=O (V)
avec un composé de formule VI
M-B (VI)
dans laquelle M représente l'hydrogène ou le lithium.

7. Procédé de préparation des composés de formule I dans laquelle W = O ou de leurs sels selon la revendication 1, caractérisé par le fait que l'on fait réagir de manière connue en soi un composé de formule VII
A-SO₂-Cl (VII)
en présence d'une base forte, avec un amide de formule VIII

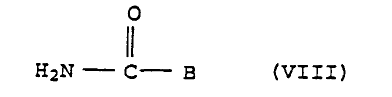

8. Procédé de préparation des composés de formule I dans laquelle W = O ou de leurs sels selon la revendication 1, caractérisé par le fait que l'on fait réagir de manière connue en soi un composé de formule IX
A-SO₂-NHT (IX)
dans laquelle T représente un métal alcalin, avec un composé de formule X
ASO₂NHal₂ (X)
dans laquelle Hal présente le chlore ou le brome, et un aldéhyde de formule XI
B-CHO (XI)

9. Procédé de préparation des sulfonamides de formule générale I 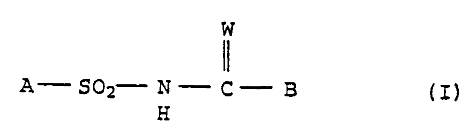 dans laquelle les symboles ont les significations indiquées dans la revendication 1 ;
et de leurs sels non polluants,
caractérisé par le fait que l'on fait réagir de manière connue en soi un composé de formule I dans laquelle W = O avec le composé de formule XII

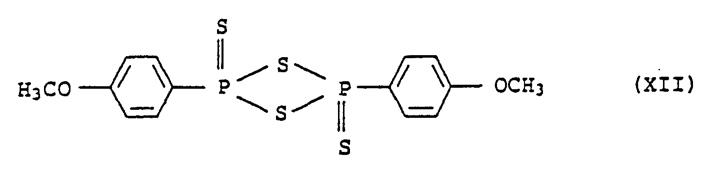

10. Procédé de préparation des composés de formule I dans laquelle W = O ou de leurs sels selon la revendication 1, caractérisé par le fait que l'on fait réagir de manière connue en soi un composé de formule XIII 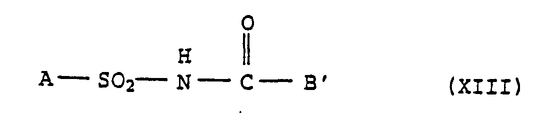 dans laquelle l'hétérocycle B' porte, fixé sur le carbone, un substituant qui fait fonction de groupe éliminable, avec un réactif nucléophile.

11. Produit herbicide contenant un sulfonamide de formule I selon la revendication 1 ou l'un de ses sels et des additifs inertes usuels.

12. Produit permettant d'agir sur la croissance des végétaux, qui contient un sulfonamide de formule I selon la revendication 1 ou l'un de ses sels avec des additifs inertes usuels.

13. Procédé pour combattre les croissances de végétaux indésirables, caractérisé par le fait que l'on fait agir un sulfonamide de formule I selon la revendication 1 ou l'un de ses sels sur les végétaux et/ou leur habitat.

14. Procédé pour agir sur la croissance des végétaux, caractérisé par le fait que l'on fait agir un sulfonamide de formule I selon la revendication 1 ou l'un de ses sels sur les végétaux et/ou leur habitat.
